# EUROPEAN PATENT SPECIFICATION

(11) **EP 1 539 224 B1**
(45) Date of publication and mention of the grant of the patent: **28.11.2007**
(21) Application number: 03797125.6
(22) Date of filing: 18.09.2003
(51) Int. Cl.: A61K 38/47, A23K 1/17, A61P 31/04

(54) **ANTIMICROBIAL COMPOSITION AND METHOD FOR USE**
ANTIMIKROBIELLE ZUSAMMENSETZUNG UND ANWENDUNGSVERFAHREN
COMPOSITION ANTIMICROBIENNE ET SA METHODE D'UTILISATION

(30) Priority: 18.09.2002 CA 2404356
(43) Date of publication of application: 15.06.2005
(73) Proprietor: Neova Technologies Inc., Abbotsford, BC V2T 6K8 (CA)
(72) Inventor: SMITH, Stephen, R., Abbotsford, British Columbia V3G 1J4 (CA); RICHIE, Stuart, J., Abbotsford, British Columbia V4X 2N4 (CA); ZHANG, Guopeng, Langley, British Columbia V1M 1E6 (CA)
(74) Representative: Ratcliffe, Susan Margaret
(86) International application number: PCT/CA2003/001359
(87) International publication number: WO 2004/026334

(56) References cited:
- EP-A- 0 466 244
- EP-A- 0 955 061
- PATENT ABSTRACTS OF JAPAN vol. 011, no. 371 (C-462), 3 December 1987 (1987-12-03) & JP 62 145025 A (NIPPON KAYAKU CO LTD), 29 June 1987 (1987-06-29)

## Description

### Field of the Invention

The present invention relates to a composition for suppressing the growth of microorganisms in the gut of livestock, and more particularly, to an antimicrobial composition that can be administered to livestock as an additive to water or feed to suppress the growth of pathogenic microorganisms in the gut.

### Background of the Invention

Bacteria cause numerous intestinal diseases in livestock. These types of bacteria fall into the general class of pathogenic enteric bacteria. One such bacterium that causes intestinal disease is *Clostridium perfringens,* which causes necrotic enteritis ("NE") in poultry and *Clostridium perfringens* enteritis ("CPE") in swine. *Escherichia coli,* another common enteric pathogen, causes disease in many types of animals, such as diarrheal disease in piglets. Every year NE, CPE and diarrheal diseases impose significant financial losses to the bird and pig farming industries. In addition to *Cl. perfringens* and *E. coli,* various serotypes of *Salmonella* cause diseases in animals which can be transmitted to humans through the consumption of animal food products.

Cases of NE and CPE have been reported in almost all areas of the world and play a significant economic role in the viability of the poultry and swine industries. For example, the annual worldwide loss due to NE infections is estimated to be more than two billion dollars US.

NE was first described as a domestic avian disease in 1961. *Clostridium perfringens* is found naturally occurring in the avian gut. Under favorable conditions, however, *Clostridium perfringens* can multiply quickly and release toxins, which cause gross lesions consisting of large areas of necrosis in the lining of the lower small intestine. In some cases the bacteria can also affect the caeca and the liver. Favourable conditions leading to the proliferation of Clostridium perfringens can be induced by intestinal stresses caused by dietary risk factors and other factors such as coccidiosis etc., acting as predisposing factors for the disease. The classical form of NE tends to occur as an outbreak, principally affecting birds between the age range of two to five weeks and is characterized by an acute course of loss of appetite, depression, ruffled feathers, diarrhea and decreased weight gain which may also be followed by sudden death. The mortality rate in untreated flocks can reach 10% or more. Similarly in swine, Clostridium perfringens type A and type C cause diarrhea, decreased weight gain and sometimes death in acute forms.

Bacterial diarrhea otherwise known as diarrheal disease, has generally become an economically important disease in the livestock industry and more particularly in swine, as a result of increasing intensification of farrowing management-a trend towards large herds and early weaning. The diarrheal disease caused by enterotoxigenic Escherichia coli is the most common enteric disease encountered in neonatal pigs. At the onset of the disease Escherichia coli colonizes in the gut by adhering to the epithelium of the small intestine. Once the E. coli colonizes, the bacteria then produce toxins that cause gastro- intestinal disorders. The process of infestation can occur in both neonatal and post-weaning piglets. The post-weaning infection of Escherichia coli, however, is a major cause of economic loss in the swine industry due to reduced growth rates and increased mortality rates. One of the most common causes of post- weaning mortality on pig farms, killing 1.5-2% of pigs weaned, is diarrheal disease.

A variety of antibiotics such as virginiamycin and bacitracin have been used to control and prevent Clostridium perfringens and other related diseases. These antibiotics are administered to various avian and swine populations by adding it to their feed. Synergistic antibacterial are discussed in EP466244 together with an antimycotic component. The continued development of resistance to and against feed grade antibiotics, however, has caused a setback in the prevention and control of the above-mentioned diseases. Further, in July 1999, the European community banned the use of feed-grade antibiotics, including virginiamycin and bacitracin. A similar action may soon be undertaken in the rest of the world due to the increasing public awareness of the negative impacts of antibiotic use and its affect on the environment and human health. One of the most significant problems associated with the reduction and elimination of antibiotics for use in poultry and swine will be the increase in incidences of NE, along with a potential increase in incidences of CPE and diarrheal disease. Moreover, other sectors of the animal industry will be immediately threatened due to the prevalence of various diseases after antibiotic withdrawal. A withdrawal will also result in a decrease in the safety of the food that is consumed by humans as animal food products. For the foregoing reasons there is a need for a cost-effective alternative to reduce the incidence of or to prevent gastrointestinal diseases, such as, NE, CPE and diarrheal disease in animals and more particularly in avian and swine populations. Others have discussed the use of materials other than traditional antibiotics such as egg derived antibodies, for example freeze dried egg yolks or liquid eggs are discussed in EP 955061, while JP 62145025 mentioned the use of albumen as an antiviral.

### Summary of the Invention

It is therefore an object of the present invention to provide an improved antimicrobial composition for administering the same to assist in controlling enteric pathogenic infection in livestock and more particularly in avian and swine populations.

Thus, according to the invention, there is provided a new agent for suppressing the growth of enteric pathogens in the gut of livestock as set out in claim 1. In particular the antimicrobial composition includes lysozyme in combination with an antimicrobial substance and a sequestering agent to reduce the growth of and diseases or epidemiological significant effects caused by Clostridium perfinigens, E coli and Salmonella, and to address at least some of the problems identified above.

The antimicrobial composition according to embodiments of the invention suppress various types of bacteria that may be present in vivo in the avian and swine guts which may affect the animals' health and can lead to complications such as decreased weight gain and death. More particularly, the antimicrobial composition and method relate to a water or feed additive that can be administered to livestock to inhibit the growth of *Clostridium perfringens,* which causes NE in poultry and CPE in swine. The use of such a water or feed additive may also inhibit the growth of *E*. *coli* which causes diarrheal disease in swine and may also inhibit the growth of *Salmonella* Typhimurium *and Salmonella* Mbandaka, including other enteric pathogens present in the avian and swine guts.

According to an embodiment of the invention, there is provided a antimicrobial composition including:
(a) a cell wall lysing substance or its salt;
(b) an antimicrobial substance; and
(c) a sequestering agent.

In a particular case, the ratio of the cell wall lysing substance or its salt, the antimicrobial substance and the sequestering agent is 2:5:3 by weight.

In avian and swinepopulations, particular enteric pathogens targeted include members of the following families of bacteria: *Clostridium perfringens, Escherichia coli, Salmonella* Typhimurium *and Salmonella* Mbandaka.

In another particular case the cell wall lysing substance or its salt may be lysozyme.

In another particular case the antimicrobial substance may be dried egg powder and /or albumen.

In further particular case, the sequestering agent may be an organic acid and/or a metal-chelator and may be selected from disodium ethylenediaminetetraacetate (EDTA), citric acid or chitosan.

In another particular case the composition further comprises a lantibiotic which may be nisin.

In another particular case the antimicrobial composition is in powdered or aqueous solution form.

In another particular case the antimicrobial composition is administered as a feed additive.

The administration of the antimicrobial composition is to suppress the incidence of or treat necrotic enteritis,Clostridium perfringens enteritis and diarrheal disease.

In another particular case the dried egg powder may suppress the growth of additional microbes such as molds and viruses in the livestock gut. Further, the dried egg powder may also suppress additional enzymes such as proteases and lipases in the livestock gut.

In a further particular case the antimicrobial composition can be administered in aqueous form by mixing the antimicrobial composition with drinking water for the livestock where the antimicrobial composition may have a final concentration of approximately 100 to 200 parts per million.

In yet another particular case the antimicrobial composition can be administered as a food additive where the antimicrobial composition may have a final concentration of approximately 100 to 200 parts per million.

There is also provided a method for suppressing the growth of enteric pathogens in the gut of livestock and the incidence of diseases related thereto, the method including administering an antimicrobial composition to the livestock, the antimicrobial composition comprising:
(a) a cell wall lysing substance or its salt ;
(b) an antimicrobial substance;
(c) a sequestering agent; and
(d) a lantibiotic.

In a particular case the ratio of the cell wall lysing substance or its salt, the antimicrobial substance, the sequestering agent and the lantibiotic are approximately 50: 150: 50: 20 by weight.

Further, there is provided an antimicrobial composition for suppressing the growth of enteric pathogens in the gut of livestock and the incidence of diseases related thereto, the antimicrobial composition comprising:
(a) a cell wall lysing substance or its salt ;
(b) a antimicrobial substance;
(c) a sequestering agent; and
(d) a lantibiotic.

In a particular case the enteric bacterial pathogens targeted include members of the following families of bacteria: Clostridium perfringens, Escherichia coli, Salmonella Typhimurium and Salmonella Mbandaka.

In another particular case the cell wall lysing substance or its salt may be lysozyme.

In another particular case the antimicrobial substance may be dried egg powder and/or albumin.

In another particular case the sequestering agent may be an organic acid and/or metal-chelator and may be selected from disodium ethylenediamine tetraacetate (EDTA), citric acid or chitosan.

In another particular case the lantibiotic may be nisin.

In another particular case the antimicrobial composition may be in powdered or aqueous solution form.

In another particular case the antimicrobial composition may be a feed additive.

Other aspects and features of the present invention will become apparent to those of ordinary skill in the art upon review of the following description of embodiments of the invention in conjunction with the accompanying figures.

### Brief Description of the Drawings

The embodiments of the present invention shall be more clearly understood with reference to the following description of the preferred embodiments and to the accompanying figures, in which:

Fig. 1 illustrates the minimal inhibitory concentration (MIC) of lysozyme against *Cl. perfringens,*

Fig. 2 illustrates the MIC of a blend containing lysozyme, albumen and citric acid at a ratio of 50:100:50 against *Cl. perfringens;*

Fig. 3 illustrates the inhibition of *Cl. perfringens* by lysozyme, disodium EDTA and dried egg powder at a ratio of 2:3:5 (Inovapure^{®} Plus 532 from Canadian Inovatech Inc., Abbotsford, B.C., Canada) at concentrations of 100 and 200 ppm;

Fig. 4 illustrates the MIC of Inovapure^{®} Plus 532 against *E. coli*;

Fig. 5 illustrates the MIC of Inovapure^{®} Plus 532 against *Salmonella* Typhimurium;

Fig. 6 illustrates the MIC of Inovapure^{®} Plus 532 against *Salmonella* Mbandaka;

Fig. 7 illustrates the fractional inhibitory concentration (FIC) of lysozyme and nisin against *Cl. perfringens;* and

Fig. 8 illustrates the effect of lysozyme, nisin, citric acid and albumen against *Cl. perfringens.*

### Description of the Invention

In the control or suppression of bacteria in human food products, the use of cell lysing substances to break down the cell walls of the bacteria is known. For example, the enzyme commonly known as lysozyme (also know as EC 3.2.1.17 or muramidase) naturally occurs in several mammalian secretions such as milk, saliva and tears. In industry, lysozyme is extracted from hen egg whites due to its natural abundance, including approximately 3% of the protein. Lysozyme is a 14.6 kDa single peptide protein that can lyse bacterial cells by cleaving the β(1-4) glycosidic linkage between *N*-acetylmuramic acid and *N-*acetylglucosamine in the peptidoglycan layer. Lysozyme has an extremely high isoelectric point (>10) and consequently is highly cationic at a neutral or acid pH. In solution, lysozyme is relatively stable at a low pH. It is also active over the temperature range of 1°C to near boiling, approximately 100°C.

Lysozyme is effective against certain Gram-positive bacteria including the *Clostridium* species. By way of example, lysozyme has been used in the cheese industry as a bio-protectant for more than 20 years to prevent butyric spoilage, which causes the late blowing of semi-hard cheeses, by *Clostridium tyrobutyricum.* For a discussion of this topic see, Identification of *Clostridium tyrobutyricum* as the causative agent of late blowing in cheese by species-specific PCR amplification, N Klijn, F F Nieuwenhof, J D Hoolwerf, C B van der Waals, and A H Weerkamp, Appl Environ Microbiol. 1995 August; 61 (8): 2919-2924.

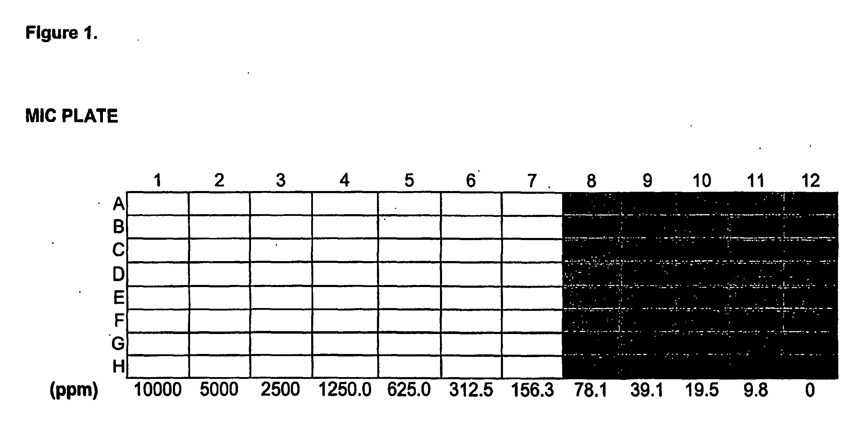

In an embodiment of the invention, an antimicrobial composition containing lysozyme may also act more particularly as an antibacterial to control and prevent enteric pathogenic bacterial related diseases in livestock by administering the antimicrobial composition to livestock through their feed. In a pilot study, it has been shown that lysozyme is effective in inhibiting the pathogenic bacteria *Clostridium perfringens.* Figure 1 illustrates the data obtained from the minimal inhibitory concentration ("MIC") plates for lysozyme against *Clostridium perfringens.* Here, lysozyme shows efficacy for inhibiting *Clostridium perfringens* at a dose of between 100 and 200 ppm. Lysozyme may be added to animal feed and more particularly, bird and swine feed, at this concentration in order to control and prevent the enteric pathogenic effects of *Clostridium prefringens,* along with other bacterial growth.

Currently, antibiotic usage costs approximately USD$3 to USD$5 per metric ton of feed (MTF) in order to maintain efficacy. To maintain the same equivalent efficacy using lysozyme at 200 ppm it would cost approximately USD$20/MTF. While the cost of lysozyme may increase the overall cost of treating animals, the use of lysozyme has the advantage of reducing or eliminating the need for conventional antibiotics, so that any issues with respect to antibiotic resistance, which may arise in a given population as described above, can be overcome.

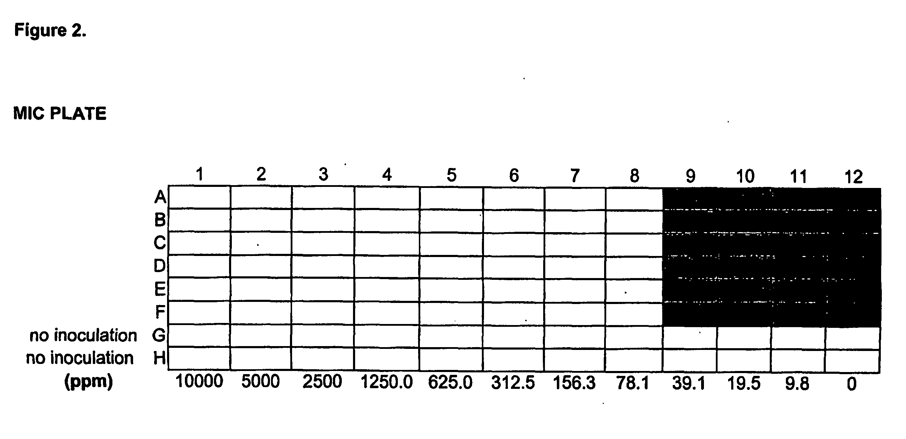

In an embodiment of the invention, an antimicrobial composition containing a cell wall lysing substance, such as lysozyme, an antimicrobial substance, preferably dried egg powder/albumin and a sequestering agent or metal-chelating agent are administered to livestock to reduce the incidence of the above mentioned bacterial related diseases. In a pilot study, it has been shown that by adding dried egg powder and a sequestering/metal-chelating agent, such as disodium ethylene diamine tetraacetic acid (disodium EDTA), citric acid or chitosan, to lysozyme, there is an increased efficacy for inhibiting *Clostridium perfringens.* It is anticipated that this is due to synergistic effects between lysozyme, dried egg powder and the sequestering/metal-chelating agent. Experimental data relating to the synergies between lysozyme, citric acid and albumen are shown in **Figure 2. Figure 2** illustrates the MIC plates of *Clostridium perfringens* inoculated with a 100 times dilution of an overnight culture. In this pilot study, the mixture (Blend 1) contains lysozyme, albumen and citric acid in a ratio of 1:3:1.

**Figure 2** illustrates that there is an efficacy for inhibiting *Clostridium perfringens* at approximately 50ppm when using the above-described Blend 1 on MIC plates. It should be noted that, although the relative amount of lysozyme used in the present pilot study decreased compared to the concentration used in the pilot study of Figure 1, the efficacy of the composition increased. It is also important to note that lysozyme represents only a fraction of Blend 1 used in the presently described pilot study (50 ppm) that led to the generation of the data illustrated in Figure 2.

An advantage of this embodiment of the invention is the relative low costs of both albumen and citric acid as compared to lysozyme. Given current prices, Blend 1 would cost approximately USD$3/MTF at 50ppm in order to be effective in controlling *Clostridium perfringens.*

There is also some indication that the sequestering agent/metal-chelating agent, i.e. citric acid, may also help to prevent the growth of Gram negative pathogens such *as Escherichia coli* and *Salmonella.* In a further pilot study using Blend 1, it was found that there was an increase in antimicrobial activity against Gram negative pathogens. It has been suggested that the reason for this is that the sequestering/metal-chelating agent works as an anti-oxidant and is synergistic with lysozyme in these situations. By keeping the individual gut of livestock, such as birds and pigs, more acidic, the overall effectiveness of inhibiting *Clostridium perfringens* and other pathogens may be increased.

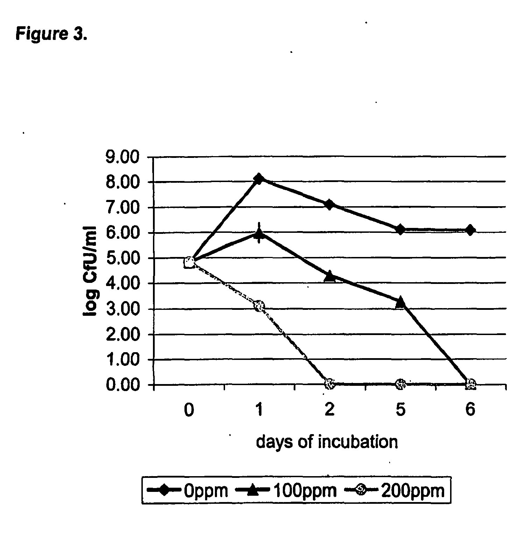

In another embodiment of the invention, an antimicrobial composition containing lysozyme, disodium EDTA and dried egg powder may be added to the feed of livestock and more particularly, poultry and /or swine in order to control and suppress bacteria and bacterial related diseases. In a further pilot study, it has been shown that a blend Inovapure^{®} Plus™ 532 from Canadian Inovatech Inc., Abbotsford, B.C., Canada is synergistic and successful at controlling *Clostridium perfringens.* The results from the pilot study are illustrated in Figure 3.

In this pilot study, *Clostridium perfringens* was inoculated at 10⁵ CFU/ml in LB medium supplemented with 0.15% NaCl. Both 100 ppm and 200 ppm of the Inovapure^{®} Plus 532 successfully inhibited the growth of the *Clostridium perfringens.* Furthermore a dose response was evidenced by the fact that the 200 ppm solution was more successful.

One of the benefits of using disodium EDTA as the metal-chelator is that it is an approved ingredient for use in animal feed in both Canada and the United States. In addition, EDTA itself has antimicrobial properties because it limits the availability of cations in a solution. In limiting the cations in a solution EDTA complexes with the cations that act as salt bridges between membrane macromolecules, such as lipopolysaccharides causing the bacterial cell membranes to become unstable and lyse.

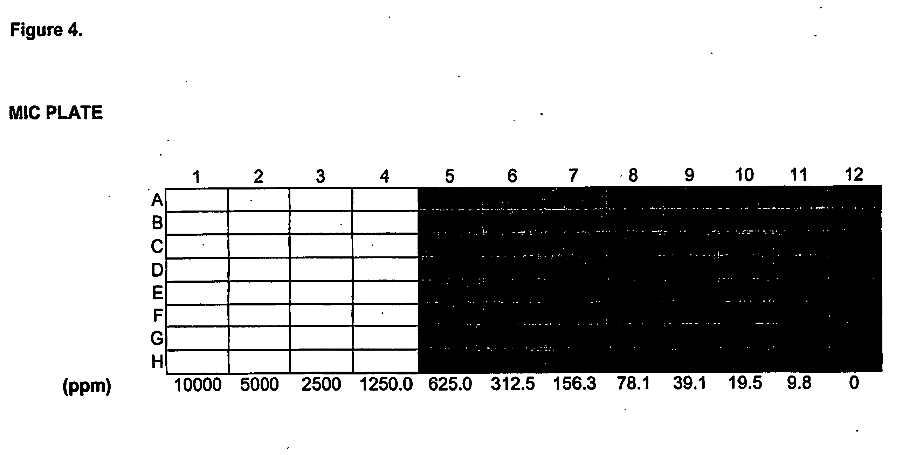

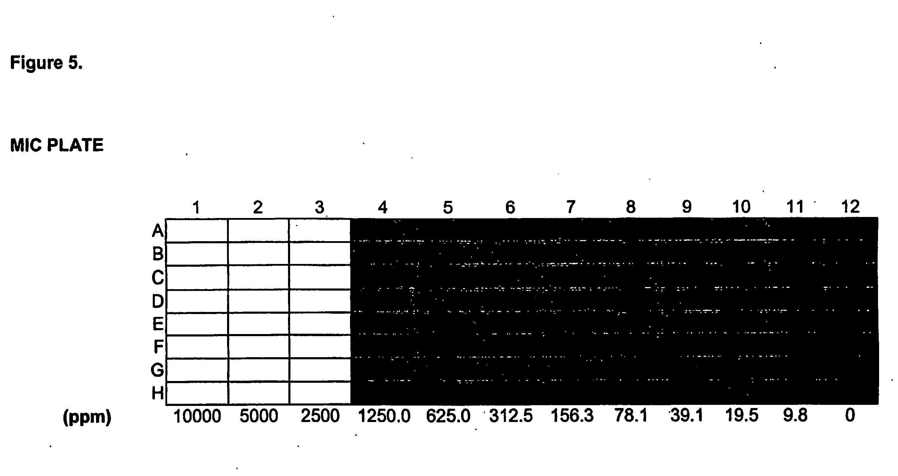

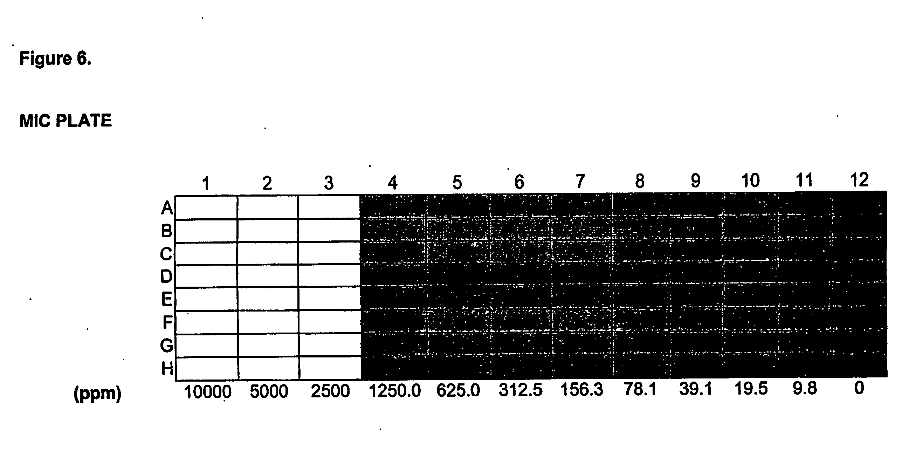

In yet another embodiment of the invention, synergistic effects of lysozyme and EDTA have been observed on other types of microorganisms, such as, Gram-positive bacteria, more particularly, Staphylococcus aureus and various Gram negative bacteria. Lysozyme and EDTA have also been observed to be effective on certain types of fungi. **Figures 4, 5** and **6** illustrate the MIC of Inovapure^{®} Plus 532 against clinical isolates of *Salmonella* Typhimurium, *Salmonella* Mbandaka, and *Escherichia coli.*

In the pilot study that led to the generation of **Figures 4, 5 and 6,** the MIC plates of *E. coli, Salmonella* Typhimurium, and *Salmonella* Mbandaka were inoculated with a 10,000 times dilution of an overnight culture. **Figure 4** illustrates that the efficacy for inhibiting *Escherichia coli* with Inovapure^{®} Plus 532 was approximately 1250 ppm while the cultures of *Salmonella* were both inhibited at approximately 2500 ppm.

A further benefit obtained from the use of lysozyme and EDTA is that it is known that both lysozyme and EDTA have a direct inhibitory effect on the enzymatic activity of phospholipase C (or the alpha toxin produced by *Clostridium perfringens)* which causes intestinal lesions, a further benefit of the embodiment of the invention.

The use of dried egg powder, which includes various egg components, provides the added benefit that other native components of hen egg whites have been shown to have. Native components of hen egg whites have been shown to work well in inhibiting numerous bacteria, yeasts, molds and viruses, as well as proteases and lipases. For a discussion of various ovo-antimicrobials see, for example, Natural Food Antimicrobial Systems, ed. A. S. Naidu, pp.211-226. New York: CRC Press, Inc, and Lineweaver, H., and C. W. Murray. 1947. Identification of the trypsin inhibitor of egg white with ovomucoid. J. Biol. Chem. 171, 565-581. In addition, the synergies of the dried egg proteins also serve to inhibit the toxins that are produced by *Clostridium perfringens.* For a discussion of these various effects including the effect of immunoglobulin Y (IgY) derived from dried egg proteins see, for example, Erhard, M.H., Gobel, E., Lewan, B., Losch, U., Stangassinger, M., 1997. Systemic availability of bovine immunoglobulin G and chicken immunoglobulin Y after feeding colostrum and egg powder to newborn calves. Archives of Animal Nutrition, 50(4), 369-380; Jin, I.Z., Baidoo, S.K., Marquardt, R.R. Frohlich, A.A., 1998. In vitro inhibition of adhesion of enterotoxigenic Escherichia coli K88 to piglet intestinal mucus by egg-yolk antibodies. Immunology and Medical Microbiology, 21, 313-321; Marquardt, R.R., 2000. Control of Intestinal diseases in pigs by feeding specific chicken egg antibodies. In: Egg nutrition and biotechnology, Sim, J.S., Nakai, S., Guenter, W., (eds). CABI Publishing, 289-299; O'Farrelly, C., Branton, D., Wanke, C.A., 1992. Oral ingestion of egg yolk immunoglobulin from hens immunized with an enterotoxigenic Escherichia coli strain prevents diarrhea in rabbits challenged with the same strain. Infection and Immunity, 60(7), 2593-2597.

In another embodiment of the invention, a composition further containing a lantibiotic may be formed to also suppress gram-positive bacterial growth and related diseases in livestock and more particularly, in avian and swine populations. In a further pilot study, it has been shown that by adding a lantibiotic, such as nisin, to lysozyme, there is an increased efficacy for inhibiting *Clostridium perfringens* due to synergistic effects between the lysozyme and the lantibiotic.

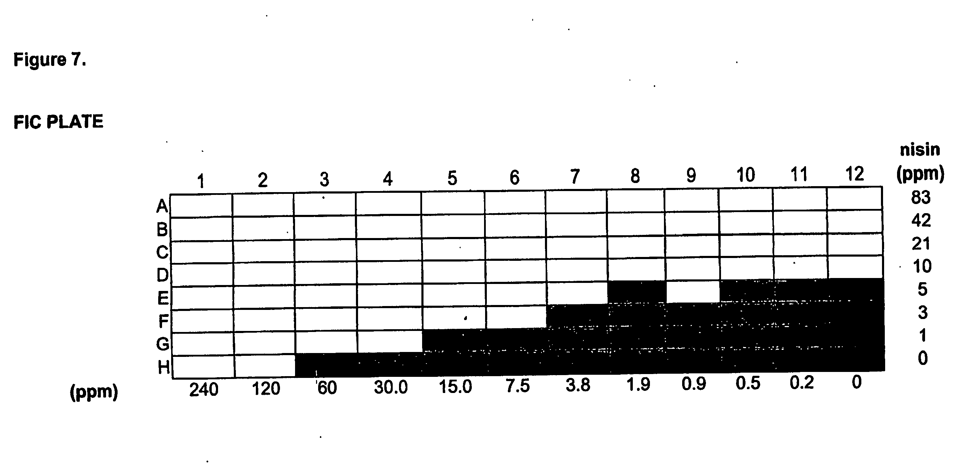

Figure 7 illustrates experimental data relating to the synergies between nisin and lysozyme obtained during this pilot study. Fractional inhibitory concentration ("FIC") plates of *Clostridium perfringens* were inoculated with a 100 times dilution of an overnight culture. It should be noted that lysozyme was effective alone in this pilot study at approximately 120ppm. By increasing the amount of nisin in the pilot study to 3 ppm, only 8 ppm of lysozyme was required to achieve a bactericidal effect on *Clostridium perfringens.*

In another embodiment of the invention, an antimicrobial composition containing lysozyme, powdered egg and/or albumen, a sequestering/metal-chelating agent and a lantibiotic can be administered to livestock to suppress bacterial growth and bacterial related diseases in livestock and more particularly in avian and swine populations. Preferably, the composition is added to the water or feed of an avian and /or swine population to prevent enteric bacterial related diseases. In a further pilot study, it was found that a composition containing a lantibiotic, such as nisin, lysozyme, albumen and a sequestering/metal-chelating agent, such as citric acid, has a high efficacy for inhibiting *Clostridium perfringens* due to the synergies between lysozyme, nisin, albumen and citric acid.

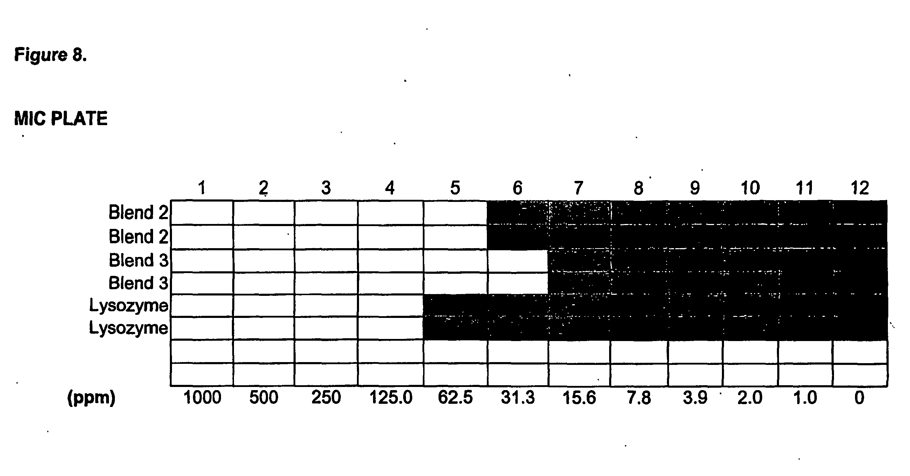

**Figure 8** illustrates experimental data relating to the synergies between nisin, lysozyme, citric acid and albumen as determined from the pilot study. Figure 8 shows MIC plates of *Clostridium perfringens* inoculated with a 100 times dilution of an overnight culture. In the pilot study two blends of the antimicrobial composition was used (Blend 2 and Blend 3). Blend 2 and Blend 3 contained lysozyme, nisin, citric acid and albumen in the ratios of 33:17:50:150 and 50:20:50:150 (Inovapure® Plus), respectively. Figure 8 shows that the effective dosage of the combination of Blend 3, against *Clostridium perfringens,* is approximately 20 ppm. Thus, by adding nisin, the effective dosage decreased from approximately 50ppm (as seen in Blend 1 Figure 2) to 20ppm. Again, the amount of lysozyme in the mixture represents only a portion of the total antimicrobial composition.

An additional advantage of this embodiment of the invention is that a blend of lysozyme, nisin, citric acid and albumen for maintaining efficacy by inhibiting *Clostridium perfringens* at 20ppm currently costs approximately less than $1 USD/MTF.

In a more recent cage study, broiler chicks of a homogenous flock were used. Four hundred birds were used in the study and it was conducted over 27 days. All birds used in the study received a routine vaccination administered in-ovo, at the hatchery. No concomitant drug therapy was used during the pilot study. The pilot study was based on a randomized complete block design. Eight cages per treatment were used and five treatments in each block were administered.

The treatments were as follows:
- 1: Nonmedicated, Nonchallenged
- 2: Nonmedicated, Challenged
- 3: Inovapure™ Plus 50 mg/kg
- 4: Inovapure™ Plus 100 mg/kg
- 5: BMD (bacitracin methylene disalicylate) 50 g/t

The mixture of the antimicrobial composition in treatment 3 and 4 was Inovapure^{®} Plus as described above.

On the fifth day of testing the birds were placed in groups. The weight per cage for each group was kept as uniform as possible. Additional weighting of each bird of each group took place on day 14 and 27 of the pilot study.

The carcasses of the birds that died during the trial were taken for lesion scoring, by way of necropsy by an investigator. The purpose of the necropsy was to determine the most probable cause of death or morbidity. NE was diagnosed as the cause of mortality if intestinal lesions were noted.

The feed that was administered to the birds during the study was a non-medicated starter and grower basal feed. The diets were set forth as starter (Day 0 to 18) and grower (Day 18 to 27). These timelines were considered to represent local industry diets. In addition, the feed consumption of the birds was monitored.

On day 14, all birds except those in treatment 1 were orally inoculated with a mixed inoculum of *E. acervulina* and *E. maxima* oocytes. The oocytes provided a light coccidial challenge to stimulate *Clostridium* proliferation in the gut and intestines of the birds. Starting on Day 18 all of the birds except treatment 1 were orally gavaged with a broth culture of C. *perfringens* at approximately 10⁸ cfu/ml. The birds were then administered a fresh broth culture daily for three days on Days 18,19 and 20.

On Day 22 two birds from each cage were randomly selected, sacrificed and examined for the presence of NE lesions. If there were lesions present they were scored. The scoring was based on a scale of 0 to 3, with 0 being normal and 3 being the most severe. On Day 27 the remaining birds were sacrificed and lesion scored.

The following table sets out the results from the cage study:

| | | Feed | Feed | Wt Gain (Kg) | | | | Lesion Score (0 to 3) | |
|---|---|---|---|---|---|---|---|---|---|
| | Treatment | Consum. | Conv. | D5 to D14 | D5 to D27 | D14to D27 | NE Mort. | Day 22 | Day 27 |
| 1. | Nonmedicated, Nonchallenged | 15.769 | 1.633 | 0.247 | 1.048 | 0.800 | 0 | 0.0 | 0.0 |
| 2. | Nonmedicated, Challenged | 13.670 | 1.775 | 0.254 | 0.882 | 0.629 | 10 | 0.2 | 0.7 |
| 3. | Inovapure® Plus 50 mg/kg | 13.667 | 1.656 | 0.252 | 0.941 | 0.689 | 5 | 0.1 | 0.4 |
| 4. | Inovapure® Plus 100 mg/kg | 13.765 | 1.636 | 0.251 | 0.965 | 0.714 | 4 | 0.1 | 0.2 |
| 5. | BMD 50 g/t | 14.000 | 1.659 | 0.252 | 0.946 | 0.694 | 2 | 0.0 | 0.3 |

Several significant findings can be deduced from the above cage study. Firstly, there was a significant reduction in the mortality rate of the birds where the antimicrobial composition was administered. In addition, there was a significant reduction in intestinal lesion development in the birds in both treatments 3 and 4. There was also a dose response elicited in the test subjects as can be observed between treatment 3 and 4. There was also a noticeable weight gain observed in both treatments where the antimicrobial composition was administered. Further to the weight gain experienced by the birds, there was a significant decrease in the feed conversion ratio (FCR) [the average feed intake /the average bird weight]. Given the above, the antimicrobial composition has a similar antimicrobial/growth promoting efficacy to that of the antibiotic BMD.

A further floor pen study was conducted in which an antimicrobial composition was administered including Inovapure^{®} Plus 532. The Applicant undertook the pilot study in order to evaluate the effect of dietary Inovapure® Plus 532 at 100 and 200 ppm on the performance of broiler chickens grown in a floor pen environment, challenged with *Clostridium perfringens* type A.

The parameters of the experiment were substantially similar to the above described cage study. This floor pen study was conducted in a randomized complete block design, using a single flock type, the standard vaccinations were administered in-ovo at the hatchery and the diets of the birds contained no anticoccidial or antibiotic, other than those prescribed in the experimental design. The study was carried out over 41 days.

The antimicrobial composition, as described above, was fed to the birds continuously for the entire grow-out. The antimicrobial composition was mixed with a complete diet at an inclusion rate of 0, 100, or 200 ppm. The antimicrobial composition was made into an intermediate premix by adding the entire amount required for each tonne with 5 Kg of a major ingredient, such as wheat, and thoroughly mixing by hand. The intermediate premix was then added to the entire mix and blended.

A coccidial challenge was administered to the chicks to stimulate *Clostridium* proliferation in the intestine of the birds.

The following are results obtained from the pilot study:

**Table 1 Effect of antimicrobial composition on Total Mortality**

| **Treatment** | **Mean** |
|---|---|
| 0 ppm | 9.89% |
| 100 ppm | 7.89% |
| 200 ppm | 7.18% |

**Table 2 Effect of antimicrobial composition on Necrotic Enteritis Mortality**

| **Treatment** | **Mean** |
|---|---|
| 0 ppm | 5.85% |
| 100 ppm | 3.89% |
| 200 ppm | 2.99% |

**Table 3 Main treatment effects on the growth and feed utilization performance**

| **Outcome Variable** | **Antimicrobial Dose** | | |
|---|---|---|---|
| | **0 ppm** | **100 ppm** | **200 ppm** |
| | | | |
| Mean Weight - Grower (kg) | 1.66 | 1.67 | 1.69 |
| Mean Feed Intake - Grower (kg) | 2.59 | 2.61 | 2.58 |
| Mean FCR^{*} - Grower | 1.56 | 1.56 | 1.53 |
| Mean Weight - Finisher (kg) | 2.12 | 2.12 | 2.15 |
| Mean Feed Intake - Finisher (kg) | 3.44 | 3.45 | 3.47 |
| Mean FCR* - Finisher | 1.63 | 1.63 | 1.61 |

As can be observed from Table 1 above, the average total mortality rate decreased where the antimicrobial composition was administered to the test subjects. There was also a dose response elicited with respect to the composition based on the total mortality. In the pilot study there was also a significant amount of NE that accounted for a component of the total mortality. Table 2 illustrates that by administering the antimicrobial composition, mortality caused by NE was significantly reduced. Again a dose response was observed.

In addition to the above, the feed consumption and the weight of the birds were carefully monitored in the pilot study, which resulted in the data outlined in Table 3. Table 3 illustrates that there was a decrease in the FCR, where the antimicrobial composition was administered. In both the grower and finisher classes of the birds there was also an increase in weight gain.

The method of administration of antimicrobial composition to livestock, including both avian and swine populations, is preferably via the oral route through drinking water or feed. To administer the antimicrobial composition in aqueous form, for example in drinking water, the composition may be produced in a powder form and then dissolved in the drinking water to yield a final concentration of approximately 100 to 200 ppm. To acquire the optimal dissolution the active ingredients should be selected to be readily soluble. For administration through feed, the antimicrobial composition is preferably added to a subset of the basal diet of an animal to form a premix at approximately 1 to 2% concentration. The pre-mix can then be mixed into the final feed at approximately 100 to 200 ppm. The blend should be used in starter, grower and finisher feeds.

## Claims

1. As an agent for suppressing the growth of enteric pathogens in the gut of livestock, said agent being provided as an antimicrobial composition comprising:
(a) a cell wall lysing substance or its salt ;
(b) at least one of dried egg powder and albumen; and
(c) a sequestering agent.

2. The antimicrobial composition according to claim 1, wherein the agent is also for preventing and treating gastrointestinal infection in livestock.

3. The antimicrobial composition according to claim 1 or claim 2, wherein the enteric pathogens include members of the following families of bacteria: Clostridium perfringens, Escherichia coli, Salmonella Typhimurium and Salmonella Mbandaka.

4. The antimicrobial composition according to claim 1, wherein the cell wall lysing substance or its salt is lysozyme.

5. The antimicrobial composition according to claim 1, wherein the antimicrobial composition includes both dried egg powder and albumen.

6. The antimicrobial composition according to claim 1, wherein the sequestering agent is an organic acid.

7. The antimicrobial composition according to claim 1, wherein the sequestering agent is a metal- chelator.

8. The antimicrobial composition according to claim 1, wherein the sequestering agent is selected from the group consisting of: (a) disodium ethylenediamine tetraacetate (EDTA); (b), citric acid; (c) chitosan.

9. The antimicrobial composition according to claim 1, further includes a lantibiotic.

10. The antimicrobial composition according to claim 9, wherein the lantibiotic is nisin.

11. The antimicrobial composition according to claim 9, wherein the ratio of the cell wall lysing substance or its salt, the at least one of fried egg powder and albumen, the sequestering agent and the lantibiotic, is 50:150:50:20 by weight

12. The antimicrobial composition according to claim 1, wherein the antimicrobial composition is in powder form.

13. The antimicrobial composition according to claim 1, wherein the antimicrobial composition is in aqueous solution form.

14. The antimicrobial composition according to claim 13, wherein the antimicrobial composition is water-soluble to allow the antimicrobial composition to be mixed with drinking water for administration of the livestock.

15. The antimicrobial composition according to claim 1, wherein the antimicrobial composition is a feed additive.

16. The antimicrobial composition according to claim 2, wherein the gastrointestinal infections include necrotic enteritis, Clostridium perfringens enteritis and diarrheal disease.

17. The antimicrobial composition according to claim 1, wherein the ratio of the cell walls lysing substance or its salt, the at least one of dried egg powder and albumen and the sequestering agent is 2:5:3 by weight.

18. The antimicrobial composition according to claim 1, wherein the antimicrobial composition includes dried egg powder and the dried egg powder is capable of suppressing additional microbes in the livestock gut.

19. The antimicrobial composition according to claim 18, wherein the additional microbes include moulds and viruses.

20. The antimicrobial composition according to claim 18, wherein the antimicrobial composition is dried egg powder and the dried egg powder is capable of suppressing additional enzymes in the livestock gut.

21. The antimicrobial composition according to claim 20 wherein the additional enzymes include proteases and lipases.

22. The antimicrobial composition according to any preceding claim wherein the composition further contains a lantibiotic.

23. The antimicrobial composition according to any preceding claim wherein the ratio of the cell walls lysing substance or its salt, the at least one of dried egg powder and albumen and the sequestering agent, is 2:5:3 in weight.

## Patentansprüche

1. Ein Wirkstoff zum Unterdrücken des Wachstums von enteralen Pathogenen im Viehdarm, wobei der Wirkstoff zur Verfügung gestellt wird als eine antimikrobielle Zusammensetzung umfassend:
(a) eine zellwandlysierende Substanz oder ihr Salz;
(b) mindestens eines von getrocknetem Eipulver und Eiweiß; und
(c) ein Sequestriermittel.

2. Die antimikrobielle Zusammensetzung nach Anspruch 1, wobei der Wirkstoff auch zum Verhüten und Behandeln einer gastrointestinalen Infektion im Vieh vorgesehen ist.

3. Die antimikrobielle Zusammensetzung nach Anspruch 1 oder 2, wobei die enteralen Pathogene Mitglieder der folgenden Familien von Bakterien einschließen: Clostridium perfringens, Escherichia coli, Salmonella Typhimurium und Salmonella Mbandaka.

4. Die antimikrobielle Zusammensetzung nach Anspruch 1, wobei die zellwandlysierende Substanz oder ihr Salz Lysozym ist.

5. Die antimikrobielle Zusammensetzung nach Anspruch 1, wobei die antimikrobielle Zusammensetzung sowohl getrocknetes Eipulver als auch Eiweiß enthält.

6. Die antimikrobielle Zusammensetzung nach Anspruch 1, wobei das Sequestriermittel eine organische Säure ist.

7. Die antimikrobielle Zusammensetzung nach Anspruch 1, wobei das Sequestriermittel ein Metall-Chelator ist.

8. Die antimikrobielle Zusammensetzung nach Anspruch 1, wobei das Sequestriermittel ausgewählt ist aus der Gruppe bestehend aus: (a) Dinatrium Ethylen-Diamin-Tetra-Acetat (EDTA); (b) Zitronensäure; (c) Chitosan.

9. Die antimikrobielle Zusammensetzung nach Anspruch 1, ferner einschließend ein Lantibiotikum.

10. Die antimikrobielle Zusammensetzung nach Anspruch 9, wobei das Lantibiotikum Nisin ist.

11. Die antimikrobielle Zusammensetzung nach Anspruch 9, wobei das Verhältnis von zellwandlysierender Substanz oder ihrem Salz, dem mindesten einem an getrocknetem Eipulver und Eiweiß, dem Sequestriermittel und dem Lantibiotikum 50:150:50:20 Gewichtsanteile beträgt.

12. Die antimikrobielle Zusammensetzung nach Anspruch 1, wobei die antimikrobielle Zusammensetzung in Pulverform vorliegt.

13. Die antimikrobielle Zusammensetzung nach Anspruch 1, wobei die antimikrobielle Zusammensetzung als wässrige Lösung vorliegt.

14. Die antimikrobielle Zusammensetzung nach Anspruch 13, wobei die antimikrobielle Zusammensetzung wasserlöslich ist, um es zu erlauben, dass die antimikrobielle Zusammensetzung mit Trinkwasser gemischt wird zur Verabreichung an das Vieh.

15. Die antimikrobielle Zusammensetzung nach Anspruch 1, wobei die antimikrobielle Zusammensetzung ein Futterzusatz ist.

16. Die antimikrobielle Zusammensetzung nach Anspruch 2, wobei die gastrointestinalen Infektionen nekrotische Enteritis, Clostridium perfringens Enteritis und Durchfallerkrankung einschließen.

17. Die antimikrobielle Zusammensetzung nach Anspruch 1, wobei das Verhältnis von zellwandlysierender Substanz oder ihrem Salz, dem mindestens einem von getrockneten Eipulver und Eiweiß und dem Sequestriermittel 2:5:3 Gewichtsanteile beträgt.

18. Die antimikrobielle Zusammensetzung nach Anspruch 1, wobei die antimikrobielle Zusammensetzung getrocknetes Eipulver einschließt und das getrocknete Eipulver in der Lage ist, zusätzliche Mikroben in dem Viehdarm zu unterdrücken.

19. Die antimikrobielle Zusammensetzung nach Anspruch 18, wobei die zusätzlichen Mikroben Schimmelpilze und Viren einschließen.

20. Die antimikrobielle Zusammensetzung nach Anspruch 18, wobei die antimikrobielle Zusammensetzung getrocknetes Eipulver ist und das getrocknete Eipulver in der Lage ist, zusätzliche Enzyme im Viehdarm zu unterdrücken.

21. Die antimikrobielle Zusammensetzung nach Anspruch 20, wobei die zusätzlichen Enzyme Proteasen und Lipasen einschließen.

22. Die antimikrobielle Zusammensetzung nach einem der vorhergehenden Ansprüche, wobei die Zusammensetzung weiter ein Lantibiotikum enthält.

23. Die antimikrobielle Zusammensetzung nach einem der vorhergehenden Ansprüche, wobei das Verhältnis von zellwandlysierender Substanz oder ihrem Salz, dem mindestens einem von getrockneten Eipulver und Eiweiß und dem Sequestriermittel 2:5:3 Gewichtsanteile beträgt.

## Revendications

1. Agent pour la suppression de la croissance d'organismes pathogènes entériques dans l'intestin du bétail, ledit agent étant fourni sous forme d'une composition antimicrobienne comprenant :
(a) une substance lysant les parois cellulaires ou son sel ;
(b) au moins un des agents consistant en de la poudre d'oeuf déshydratée et de l'albumine ; et
(c) un agent séquestrant.

2. Composition antimicrobienne suivant la revendication 1, dans laquelle l'agent est également destiné à la prévention ou au traitement d'une infection gastro-intestinale chez le bétail.

3. Composition antimicrobienne suivant la revendication 1 ou la revendication 2, dans laquelle les organismes pathogènes entériques comprennent des membres des familles de bactéries suivantes : Clostridium perfringens, Escherichia coli, Salmonella typhimurium et Salmonella mbandaka.

4. Composition antimicrobienne suivant la revendication 1, dans laquelle la substance lysant les parois cellulaires ou son sel est le lysozyme.

5. Composition antimicrobienne suivant la revendication 1, ladite composition antimicrobienne comprenant à la fois de la poudre d'oeuf déshydratée et de l'albumine.

6. Composition antimicrobienne suivant la revendication 1, dans laquelle l'agent séquestrant est un acide organique.

7. Composition antimicrobienne suivant la revendication 1, dans laquelle l'agent séquestrant est un agent chélatant les métaux.

8. Composition antimicrobienne suivant la revendication 1, dans laquelle l'agent séquestrant est choisi dans le groupe consistant en : (a) l'éthylènediamine-tétraacétate disodique (EDTA) ; (b) l'acide citrique ; et (c) le chitosane.

9. Composition antimicrobienne suivant la revendication 1, comprenant en outre un lantibiotique.

10. Composition antimicrobienne suivant la revendication 9, dans laquelle le lantibiotique est la nisine.

11. Composition antimicrobienne suivant la revendication 9, dans laquelle le rapport de la substance lysant les parois cellulaires ou de son sel, dudit au moins un des agents consistant en la poudre d'oeuf déshydratée et l'albumine, de l'agent séquestrant et du lantibiotique est de 50:150:50:20 en poids.

12. Composition antimicrobienne suivant la revendication 1, ladite composition antimicrobienne étant sous forme de poudre.

13. Composition antimicrobienne suivant la revendication 1, ladite composition antimicrobienne étant sous forme d'une solution aqueuse.

14. Composition antimicrobienne suivant la revendication 13, ladite composition antimicrobienne étant hydrosoluble pour permettre de mélanger la composition antimicrobienne à de l'eau de boisson pour l'administration au bétail.

15. Composition antimicrobienne suivant la revendication 1, ladite composition antimicrobienne étant un additif pour aliments pour animaux.

16. Composition antimicrobienne suivant la revendication 2, dans laquelle les infections gastro-intestinales comprennent l'entérite nécrotique, l'entérite à Clostridium perfringens et la maladie diarrhéique.

17. Composition antimicrobienne suivant la revendication 1, dans laquelle le rapport de la substance lysant les parois cellulaires ou de son sel, dudit au moins un des agents consistant en la poudre d'oeuf déshydratée et l'albumine, et de l'agent séquestrant est de 2:5:3 en poids.

18. Composition antimicrobienne suivant la revendication 1, ladite composition antimicrobienne comprenant de la poudre d'oeuf déshydratée, la poudre d'oeuf déshydratée étant capable de supprimer des microorganismes supplémentaires dans l'intestin du bétail.

19. Composition antimicrobienne suivant la revendication 18, dans laquelle les microorganismes supplémentaires comprennent des moisissures et des virus.

20. Composition antimicrobienne suivant la revendication 18, ladite composition antimicrobienne étant de la poudre d'oeuf déshydratée, la poudre d'oeuf déshydratée étant capable de supprimer des enzymes supplémentaires dans l'intestin du bétail.

21. Composition antimicrobienne suivant la revendication 20, dans laquelle les enzymes supplémentaires comprennent des protéases et des lipases.

22. Composition antimicrobienne suivant l'une quelconque des revendications précédentes, la composition contenant en outre un lantibiotique.

23. Composition antimicrobienne suivant l'une quelconque des revendications précédentes, dans laquelle le rapport de la substance lysant les parois cellulaires ou de son sel, dudit au moins un des agents consistant en la poudre d'oeuf déshydratée et l'albumine, et de l'agent séquestrant est de 2:5:3 en poids.
